(19) Europäisches Patentamt
European Patent Office
Office européen des brevets

(11) EP 4 052 784 A1

(12) **EUROPEAN PATENT APPLICATION**
published in accordance with Art. 153(4) EPC

(43) Date of publication:
**07.09.2022 Bulletin 2022/36**

(21) Application number: **20883358.2**

(22) Date of filing: **29.10.2020**

(51) International Patent Classification (IPC):
***B01F 17/42*** (2006.01) ***C08J 3/12*** (2006.01)
***C08L 67/00*** (2006.01) ***C08L 101/00*** (2006.01)
***C08L 71/02*** (2006.01) ***C12P 7/62*** (2022.01)

(52) Cooperative Patent Classification (CPC):
**C08J 3/12; C08L 67/00; C08L 71/02; C08L 101/00; C12P 7/62**

(86) International application number:
**PCT/JP2020/040643**

(87) International publication number:
**WO 2021/085534 (06.05.2021 Gazette 2021/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.10.2019 JP 2019199010**
**23.01.2020 JP 2020009334**

(71) Applicant: **Kaneka Corporation**
**Osaka-shi, Osaka 530-8288 (JP)**

(72) Inventors:
- **NISHINAKA, Tomoya**
**Takasago-shi, Hyogo 676-8688 (JP)**
- **DEGUCHI, Naoki**
**Takasago-shi, Hyogo 676-8688 (JP)**
- **HATTORI, Jun**
**Takasago-shi, Hyogo 676-8688 (JP)**
- **HIRANO, Masaru**
**Takasago-shi, Hyogo 676-8688 (JP)**

(74) Representative: **Vossius & Partner Patentanwälte Rechtsanwälte mbB**
**Siebertstraße 3**
**81675 München (DE)**

(54) **METHOD FOR PRODUCING POLYHYDROXYALKANOATE AND USE OF SAME**

(57) An objective of the present invention is to provide a production method that enables PHA (e.g. PHA powder) to be produced with high productivity. The above is achieved by providing: a method for producing a polyhydroxyalkanoate, including the steps of (a) preparing an aqueous suspension that contains a polyhydroxyalkanoate and an alkylene oxide-based dispersing agent and has a pH of not more than 7 and (b) spray-drying the aqueous suspension prepared in the step (a); and the like.

FIG. 1

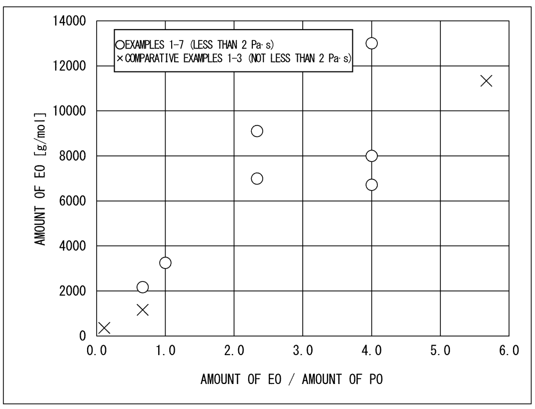


EP 4 052 784 A1

## Description

Technical Field

The present invention relates to a method for producing a polyhydroxyalkanoate and use of the polyhydroxyalkanoate.

Background Art

Polyhydroxyalkanoates (may be hereinafter referred to as "PHAs") are known to have biodegradability.

PHAs, which are produced by microorganisms, are accumulated within bacterial cells of the microorganisms. As such, in order to use PHA as a plastic, it is necessary to carry out a step of separating PHA from bacterial cells of a microorganism and purifying the PHA. The step of separating and purifying PHA involves disrupting bacterial cells of a PHA-containing microorganism or solubilizing an organism-derived component other than PHA, and then collecting the PHA from an aqueous suspension thus obtained. At this time, for example, a separating operation such as centrifugal separation, filtering, and drying is carried out. In the drying operation, a spray dryer, a fluidized bed dryer, a drum dryer, or the like is used. It is preferable to use a spray dryer in terms of simple operation.

In the past, the inventors of the present invention developed a technique in which, prior to adjustment of a pH of an aqueous suspension to not more than 7, polyvinyl alcohol (PVA) is added as a dispersing agent in order to prevent agglomeration of PHA in the aqueous suspension having a pH of not more than 7, and then the obtained aqueous suspension having a pH of not more than 7 is spray-dried (Patent Literature 1).

Citation List

[Patent Literature]

[Patent Literature 1]
International Publication No. WO 2018/070492

Summary of Invention

Technical Problem

The technique described above of Patent Literature 1 is excellent but has room for further improvement.

Thus, an objective of the present invention is to provide a production method that enables PHA (e.g., PHA powder) to be produced with high productivity.

Solution to Problem

As a result of diligent study for solving the foregoing issue, the inventors of the present invention completed the present invention after newly finding that (i) using a specific dispersing agent makes it possible to not only prevent agglomeration of PHA when adjusting a pH of an aqueous suspension to not more than 7 but also reduce or prevent adherence of PHA powder to a shaft of an extruder during processing of the PHA powder, and (ii) further adding a binder makes it possible to obtain PHA having an improved flowability.

Thus, an aspect of the present invention is a method for producing a polyhydroxyalkanoate, including the steps of: (a) preparing an aqueous suspension that contains a polyhydroxyalkanoate and an alkylene oxide-based dispersing agent and has a pH of not more than 7; and (b) spray-drying the aqueous suspension prepared in the step

(a). Another aspect of the present invention is a method for producing a polyhydroxyalkanoate, including the steps of:

(a) preparing an aqueous suspension that contains a polyhydroxyalkanoate, an alkylene oxide-based dispersing agent, and a binder and has a pH of not more than 7; and
(b) spray-drying the aqueous suspension prepared in the step (a).

Advantageous Effects of Invention

According to an aspect of the present invention, it is possible to provide a production method that enables PHA (e.g., PHA powder) to be produced with high productivity. Further, according to another aspect of the present invention,

it is possible to not only enable PHA (e.g. PHA powder) to be obtained with high productivity but also enable the PHA to have an excellent flowability.

Brief Description of Drawings

Fig. 1 is a view illustrating a relationship among an amount of EO in a dispersing agent, a ratio of the amount of the EO to an amount of PO in the dispersing agent, and a shear viscosity in Examples 1 through 7 and Comparative Examples 1 through 3.

Description of Embodiments

The following description will discuss an embodiment of the present invention in detail. Note that any numerical range expressed as "A to B" in the present specification means "not less than A and not more than B" unless otherwise stated. All literatures listed herein are incorporated herein by reference.

[Overview of the present invention]

[Aspect 1 of Present Invention]

While further advancing the study of production of PHA, the inventors of the present invention found that the above-described method of Patent Literature 1 causes a new problem, i.e., adherence of obtained PHA powder to a shaft in an extruder during processing of the PHA powder. Through analysis, the inventors of the present invention considered that the above problem was caused by PVA, which is a dispersing agent. However, not adding PVA causes PHA agglomeration in an aqueous suspension having an adjusted pH of not more than 7, and thus increases viscosity. This makes it difficult to send the aqueous suspension to a spray dryer.

In view of the above, the inventors of the present invention conducted diligent study in order to arrive at a dispersing agent that prevents agglomeration of PHA in an aqueous suspension having a pH of not more than 7 and that also prevents powder from adhering to a shaft of an extruder during processing of the powder after drying. As a result, the inventors of the present invention found that using a specific dispersing agent (specifically, an alkylene oxide-based dispersing agent) makes it possible to not only prevent agglomeration of PHA when adjusting a pH of an aqueous suspension to not more than 7 but also reduce or prevent adherence of PHA powder to a shaft of an extruder during processing of the PHA powder.

Further, while conducting the study, the inventors of the present invention found out for the first time that, surprisingly, (i) using a dispersing agent which contains not less than a certain amount of EO and in which a ratio of an amount of the EO to an amount of PO is within a specific range makes it possible to maintain a viscosity of an aqueous suspension at a low level and (ii) being thus able to use the aqueous suspension having a low viscosity makes it possible to carry out a drying step more efficiently.

Therefore, according to a production method in accordance with an embodiment of the present invention, in which a specific dispersing agent is used, it is possible to obtain PHA (e.g. PHA powder) with high productivity.

[Aspect 2 of Present Invention]

Further, the inventors of the present invention found, while further advancing their study, that using an alkylene oxide-based dispersing agent causes a new problem, i.e., a poor flowability of PHA powder after spray-drying.

In view of the above, the inventors of the present invention carried out diligent study in order to arrive at a production method that not only uses an alkylene oxide-based dispersing agent to prevent the problem of adherence of PHA powder to a shaft in an extruder but also enables the PHA powder after spray-drying to have a good flowability. As a result, the inventors of the present invention found that adding a binder to an aqueous PHA suspension together with an alkylene oxide-based dispersing agent enables PHA powder after spray-drying to have a good flowability.

Further, the inventors of the present invention found, while advancing their study, that in a case where a specific poly(3-hydroxybutyrate-co-3-hydroxyhexanoate) (P3HB3HH, also referred to as "PHBH") is used as an example of PHA, an aqueous suspension of the PHBH has a poor dispersion stability, and it thus becomes difficult to send the aqueous suspension to a spray dryer. The inventors of the present invention then found that this problem is also solvable by addition of a binder to the aqueous PHA suspension.

Thus, according to a production method in accordance with an embodiment of the present invention in which a binder is added together with an alkylene oxide-based dispersing agent, it is possible to obtain PHA (e.g. PHA powder) having an excellent flowability. Further, even in a case where the specific PHBH is used, it is possible to obtain an aqueous PHA suspension that retains a good dispersion stability. The following description will discuss, in detail, an

arrangement of a production method in accordance with an embodiment of the present invention.

[Embodiment 1]

[1. Method for producing PHA]

A method (hereinafter referred to as "the present production method") in accordance with an embodiment of the present invention for producing a polyhydroxyalkanoate includes the steps of: (a) preparing an aqueous suspension that contains a polyhydroxyalkanoate and an alkylene oxide-based dispersing agent and has a pH of not more than 7; and (b) spray-drying the aqueous suspension prepared in the step (a). That is, the present production method includes the following steps (a) and (b) as essential steps.

- Step (a): preparing an aqueous suspension that contains PHA and a dispersing agent and has a pH of not more than 7 (wherein the dispersing agent is an alkylene oxide-based dispersing agent)
- Step (b): spray-drying the aqueous suspension prepared in the step (a).

(Step (a))

In the step (a) of the present production method, an aqueous suspension that contains PHA and a specific dispersing agent and has a pH of not more than 7 is prepared. In the aqueous suspension, the PHA is present dispersed in an aqueous medium, and the dispersing agent is dissolved in the aqueous medium. Hereinafter, an aqueous suspension containing at least PHA may be abbreviated to "aqueous PHA suspension".

<PHA>

As used herein, the term "PHA" is a generic term for polymers each of which contains a hydroxyalkanoic acid as a monomer unit. A hydroxyalkanoic acid contained in PHA is not limited to any particular one, and can be, for example, 3-hydroxybutanoic acid, 4-hydroxybutanoic acid, 3-hydroxypropionic acid, 3-hydroxypentanoic acid, 3-hydroxyhexanoic acid, 3-hydroxyheptanoic acid, 3-hydroxyoctanoic acid, or the like. The polymers can be a homopolymer, or can be a copolymer containing two or more types of monomer unit.

More specifically, examples of PHA include poly(3-hydroxybutyrate) (P3HB), poly(3-hydroxybutyrate-co-3-hydroxyhexanoate) (P3HB3HH), poly(3-hydroxybutyrate-co-3-hydroxyvalerate) (P3HB3HV), poly(3-hydroxybutyrate-co-4-hydroxybutyrate) (P3HB4HB), poly(3-hydroxybutyrate-co-3-hydroxyoctanoate) (P3HB3HO), poly(3-hydroxybutyrate-co-3-hydroxyoctadecanoate) (P3HB3HOD), poly(3-hydroxybutyrate-co-3-hydroxydecanoate) (P3HB3HD), and poly(3-hydroxybutyrate-co-3-hydroxyvalerate-co-3-hydroxyhexanoate) (P3HB3HV3HH). Among these examples, P3HB, P3HB3HH, P3HB3HV, and P3HB4HB are preferable for being easily produced on an industrial scale.

Further, P3HB3HH, which is a copolymer of 3-hydroxybutyric acid and 3-hydroxyhexanoic acid, is more preferable from the following viewpoints: (i) by changing a composition ratio of repeating units, it is possible to cause a change in melting point and crystallinity and consequently in physical properties, such as Young’s modulus and heat resistance, of P3HB3HH and to enable P3HB3HH to have physical properties between the physical properties of polypropylene and the physical properties of polyethylene; and (ii) P3HB3HH is a plastic that is easily produced on an industrial scale as described above and has useful physical properties.

In an embodiment of the present invention, a composition ratio of repeating units in P3HB3HH is such that a composition ratio of a 3-hydroxybutyrate unit to a 3-hydroxyhexanoate unit is preferably 80/20 (mol/mol) to 99/1 (mol/mol), more preferably 83/17 (mol/mol) to 97/3 (mol/mol), and even more preferably 85/15 (mol/mol) to 97/3 (mol/mol) from the viewpoint of a balance between plasticity and strength. In a case where the composition ratio of the 3-hydroxybutyrate unit to the 3-hydroxyhexanoate unit is not more than 99/1 (mol/mol), a sufficient plasticity is obtained, and in a case where the composition ratio is not less than 80/20 (mol/mol), a sufficient hardness is obtained.

The step (a) preferably includes the following steps (a1) and (a2).

- Step (a1): adding a dispersing agent to an aqueous PHA suspension (wherein the dispersing agent is an alkylene oxide-based dispersing agent)
- Step (a2): adjusting a pH of the aqueous PHA suspension to not more than 7

The order of carrying out the step (a1) and the step (a2) is not particularly limited, but it is preferable to carry out the step (a2) after the step (a1), from the viewpoint of reducing or preventing agglomeration of PHA in the step (a2) and obtaining an aqueous suspension that is even more excellent in dispersion stability of PHA.

In the step (a), an aqueous PHA suspension (an aqueous PHA suspension to which the dispersing agent has

not been added) used as a starting material is not particularly limited, and can be obtained, for example, by a method including a culture step of culturing a microorganism capable of producing PHA in a cell of the microorganism and a purification step of decomposing and/or removing a substance other than PHA after the culture step.

The present production method can include, before the step (a), a step (for example, a step including the culture step and the purification step described above) of obtaining an aqueous PHA suspension (an aqueous PHA suspension to which the dispersing agent has not been added). The microorganism used in this step is not particularly limited, provided that the microorganism is capable of producing PHA in a cell of the microorganism. For example, it is possible to use a microorganism isolated from nature or a microorganism deposited at a depositary institution (for example, IFO, ATCC, or the like) for strains, or a mutant, a transformant, or the like that can be prepared from any of those microorganisms. More specific examples of the microorganism include *Cupriavidus, Alcaligenes, Ralstonia, Pseudomonas, Bacillus, Azotobacter, Nocardia,* and *Aeromonas* bacteria. Among these examples, a microorganism belonging to the genus *Aeromonas, Alcaligenes, Ralstonia,* or *Cupriavidus* is preferable. In particular, the microorganism is more preferably a strain such as *A. lipolytica, A. latus, A. caviae, A. hydrophila,* or *C. necator,* and most preferably C. *necator.*

In a case where the microorganism is inherently not capable of producing PHA or in a case where the microorganism is capable of producing only a small amount of PHA, a gene of an enzyme that synthesizes intended PHA and/or a variant of the gene can be introduced into the microorganism to obtain a transformant for use. The gene of such a PHA synthetase used in preparation of the transformant is not particularly limited, but is preferably a gene of a PHA synthetase derived from *A. caviae.* By culturing these microorganisms under an appropriate condition, it is possible to obtain bacterial cells of the microorganisms having PHA accumulated in the bacterial cells. A method of culturing the bacterial cells of a microorganism is not particularly limited, and can be, for example, a method described in Japanese Patent Application Publication Tokukaihei No. 05-93049 etc.

A PHA-containing microorganism prepared by culturing the above microorganism contains a large amount of impurity components derived from bacterial cells. As such, ordinarily, a purification step can be carried out in order to decompose and/or remove impurities that are not PHA may be carried out. In the purification step, any physical treatment, any chemical treatment, any biological treatment, or the like that can be arrived at by a person skilled in the art can be employed. For example, a purification step described in International Publication No. WO 2010/067543 is suitably employed.

An amount of impurities remaining in an end product is substantially determined by the above purification step. As such, it is preferable to minimize the impurities. Of course, depending on the purpose of use, it may be acceptable to have impurities mixed in, provided that the physical properties of the end product are not compromised. In a case where highly pure PHA is required, for example, in a case where PHA is for medical use, it is preferable to minimize impurities. At this time, an index of purification degree can be, for example, an amount of protein in the aqueous PHA suspension. The amount of protein is preferably not more than 30000 ppm, more preferably not more than 15000 ppm, even more preferably not more than 10000 ppm, and most preferably not more than 7500 ppm per weight of PHA. Means of purification is not particularly limited, and can be, for example, the foregoing publicly known method.

Note that the solvent (the "solvent" may be referred to also as "aqueous medium") contained in the aqueous PHA suspension in the present production method can be selected from water and a mixed solvent of water and an organic solvent. In the mixed solvent, a concentration of an organic solvent compatible with water is not particularly limited, provided that the concentration is not more than a water solubility of the organic solvent which is used. The organic solvent compatible with water is not particularly limited, and examples of the organic solvent include: alcohols such as methanol, ethanol, 1-propanol, 2-propanol, 1-butanol, 2-butanol, iso-butanol, pentanol, hexanol, and heptanol; ketones such as acetone and methyl ethyl ketone; ethers such as tetrahydrofuran and dioxane; nitriles such as acetonitrile and propionitrile; amides such as dimethylformamide and acetamide; dimethyl sulfoxide; pyridine; and piperidine. Among these examples, methanol, ethanol, 1-propanol, 2-propanol, 1-butanol, 2-butanol, iso-butanol, acetone, methyl ethyl ketone, tetrahydrofuran, dioxane, acetonitrile, propionitrile, and the like are preferable for being easily removable. Further, methanol, ethanol, 1-propanol, 2-propanol, butanol, acetone, and the like are more preferable for being easily available. Further, methanol, ethanol, and acetone are particularly preferable. Note that the aqueous medium contained in the aqueous PHA suspension can contain another solvent, a component derived from bacterial cells, a compound produced during purification, and the like, provided that an essence of the present invention is not compromised.

The aqueous medium contained in the aqueous PHA suspension in the present production method preferably contains water. An amount of water contained in the aqueous medium is preferably not less than 5% by weight, more preferably not less than 10% by weight, even more preferably not less than 30% by weight, and particularly preferably not less than 50% by weight.

In an embodiment of the present invention, a shear viscosity of the aqueous PHA suspension is not particularly limited. From the viewpoint of achieving an excellent flowability, however, a viscosity of the aqueous PHA suspension at a shear rate of 10 1/s is preferably 0.01 Pa·s to 2 Pa·s, and more preferably 0.02 Pa·s to 1 Pa·s. A shear viscosity of the aqueous PHA suspension is measured by a method described in Examples.

<Alkylene oxide-based dispersing agent>

The dispersing agent in the step (a) of the present production method is an alkylene oxide-based dispersing agent. In the present specification, an "alkylene oxide-based dispersing agent" may be simply referred to as a "dispersing agent".

The dispersing agent in the step (a) of the present production method is a specific dispersing agent as described above, and thus makes it possible to not only prevent agglomeration of PHA when adjusting a pH of the aqueous PHA suspension to not more than 7 but also reduce or prevent adherence of PHA powder to a shaft of an extruder during processing of the PHA powder.

In an embodiment of the present invention, the alkylene oxide-based dispersing agent is not limited to a particular one as long as it provides an effect of the present invention. It is preferable that the alkylene oxide-based dispersing agent be constituted by a block of poly(ethylene oxide) (PEO) and a block of poly(propylene oxide) (PPO), and be in a form of PEO-PPO-PEO.

As used herein, a "block of poly(ethylene oxide) (PEO)" refers to a polymer moiety formed by polymerization of ethylene oxide (EO) in a structure of the dispersing agent.

As used herein, a "block of poly(propylene oxide) (PPO)" refers to a polymer moiety formed by polymerization of propylene oxide (PO) in the structure of the dispersing agent.

In an embodiment of the present invention, in a case where a molecular weight of PEO in the dispersing agent and a ratio of the molecular weight of the PEO to a molecular weight of PPO in the dispersing agent are each within a specific range, it is possible to maintain the viscosity of the aqueous suspension and produce PHA (e.g., PHA powder) with high productivity.

In an embodiment of the present invention, it is preferable that a range of a molecular weight of PEO in the dispersing agent and a range of a ratio of the molecular weight of the PEO to a molecular weight of PPO in the dispersing agent be a combination of the following ranges.

Note that, in the present specification, a "molecular weight of PEO" may be referred to as an "amount of EO", and a "molecular weight of PPO" may be referred to as an "amount of PO".

That is, in an embodiment of the present invention, a molecular weight of PEO in the dispersing agent should be not less than 1500, and is preferably not less than 1750 and more preferably not less than 2000. Further, in an embodiment of the present invention, an upper limit of a molecular weight of PEO in the dispersing agent is, for example, not more than 30000, preferably not more than 25000, and more preferably not more than 20000.

In an embodiment of the present invention, a ratio of a molecular weight of PEO to a molecular weight of PPO in the dispersing agent should be not less than 0.5, and is preferably not less than 0.6 and more preferably not less than 0.7. An upper limit of the ratio of the molecular weight of the PEO to the molecular weight of the PPO is not more than 5.0, preferably not more than 4.8, and even more preferably not more than 4.5.

In a case where a molecular weight of PEO in the dispersing agent and a ratio of the molecular weight of the PEO to a molecular weight of PPO in the dispersing agent are within the above ranges, the dispersing agent is hydrophilic, and the number of molecules increases relative to a weight of the dispersing agent added. This makes it easier to maintain the dispersibility of the aqueous suspension.

In an embodiment of the present invention, a molecular weight of PEO in the dispersing agent is not less than 1500, and a ratio of the molecular weight of the PEO to a molecular weight of PPO in the dispersing agent is 0.5 to 5.0.

In an embodiment of the present invention, the dispersing agent includes preferably at least one, more preferably at least two, PEO blocks each having a molecular weight of not less than 750. An upper limit of the number of such PEO blocks is not particularly limited, and is, for example, not more than 4, and preferably not more than 3. In a case where the number of the PEO blocks is within the above range, the dispersing agent is hydrophilic.

In an embodiment of the present invention, a molecular weight of PPO in the dispersing agent is not particularly limited, and is, for example, not less than 500, and preferably not less than 1500. Further, in an embodiment of the present invention, an upper limit of a molecular weight of PPO in the dispersing agent is, for example, not more than 6700, and preferably not more than 6250. In a case where a molecular weight of PPO in the dispersing agent is within the above range, the dispersing agent is hydrophobic.

In an embodiment of the present invention, the number of PPO blocks in the dispersing agent is not particularly limited, provided that an effect of the present invention is exhibited. The number can be one, or can be more than one (for example, 2, 3, or 4).

In an embodiment of the present invention, the dispersing agent is, for example, a compound represented by the following formula (1):

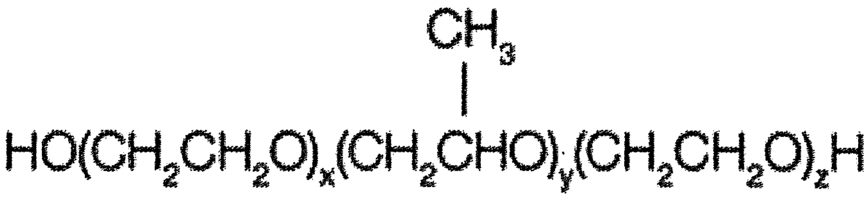

・・・(1)

where X is, for example, 17 to 340, preferably 20 to 285, and more preferably 22 to 226. In a case where X is not more than 340, the number of molecules increases relative to a weight of the dispersing agent added, and thus it is easier to maintain the dispersibility of the aqueous suspension. In a case where X is not less than 17, the dispersing agent is hydrophilic. Y is, for example, 8 to 115, preferably 10 to 110, and more preferably 24 to 107. In a case where Y is not more than 115, the dispersing agent is dissolved in water easily. In a case where Y is not less than 8, the dispersing agent is hydrophobic. Z is, for example, 17 to 340, preferably 20 to 285, and more preferably 22 to 226. In a case where Z is not more than 340, the number of molecules increases relative to a weight of the dispersing agent added, and thus it is easier to maintain the dispersibility of the aqueous suspension. In a case where Z is not less than 17, the dispersing agent is hydrophilic.

Further, in the formula (1), a sum of X and Z (may be hereinafter referred to as "X+Z") is, for example, 34 to 680, preferably 40 to 570, and more preferably 44 to 452. In a case where X+Z is not more than 680, the number of molecules increases relative to a weight of the dispersing agent added, and thus it is easier to maintain the dispersibility of the aqueous suspension. In a case where X is not less than 34, the dispersing agent is hydrophilic.

In an embodiment of the present invention, the dispersing agent is preferably a compound represented by the following formula (1):

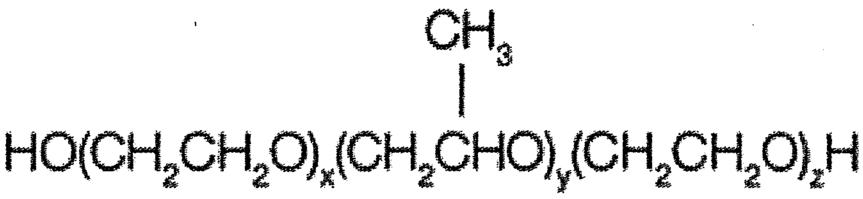

・・・(1)

where X is 17 to 340, Y is 8 to 115, and Z is 17 to 340.

The dispersing agent used in the step (a) (in particular, the step (a1)) of the present production method is not particularly limited, and can be, for example, a commercially available dispersing agent. Examples of the commercially available dispersing agent include Pluronic 10400 (manufactured by BASF), Pluronic 10500 (manufactured by BASF), Genapol PF80 (manufactured by Clariant), Unilube 70DP-600B (manufactured by NOF CORPORATION), Unilube 70DP-950B (manufactured by NOF CORPORATION), Plonon 208 (manufactured by NOF CORPORATION), Epan U105 (manufactured by Dai-ichi Kogyo Seiyaku Co., Ltd.), Epan U108 (manufactured by Dai-ichi Kogyo Seiyaku Co., Ltd.), and Epan 750 (manufactured by Dai-ichi Kogyo Seiyaku Co., Ltd.).

An amount of the dispersing agent added to the aqueous PHA suspension in the step (a) (in particular, the step (a1)) of the present production method is not particularly limited, but is preferably 0.1 parts by weight to 20 parts by weight, more preferably 0.5 parts by weight to 10 parts by weight, and even more preferably 0.75 parts by weight to 5 parts by weight relative to 100 parts by weight of the PHA contained in the aqueous suspension. In a case where the amount of the dispersing agent added is within the above range, the dispersion stability of the PHA in the aqueous PHA suspension is further improved, so that spray-drying tends to be more efficiently carried out.

<Others>

The aqueous PHA suspension (the aqueous PHA suspension to which the dispersing agent has not been added) that has not been subjected to the step (a) of the present production method ordinarily has a pH of more than 7 by being subjected to the above purification step. As such, the step (a) (in particular, the step (a2)) of the present production method is carried out to adjust the pH of the aqueous PHA suspension to not more than 7. A method of adjusting the pH is not particularly limited. For example, the pH can be adjusted by addition of an acid or by other methods. The acid is not limited to a particular one, and can be selected from an organic acid and an inorganic acid. The acid can have volatility or not have volatility. More specifically, examples of the acid include sulfuric acid, hydrochloric

acid, phosphoric acid, and acetic acid.

An upper limit of the pH of the aqueous PHA suspension adjusted in the above adjusting step is not more than 7, preferably not more than 5, and more preferably not more than 4, from the viewpoint of reducing coloring of PHA when the PHA is heated and melted and from the viewpoint of ensuring the stability of a molecular weight during heating and/or drying. A lower limit of the pH is preferably not less than 1, more preferably not less than 2, and even more preferably not less than 3, from the viewpoint of acid resistance of a container. In a case where the pH of the aqueous PHA suspension is not more than 7, obtained PHA is reduced in coloring during heating and melting of the PHA, and a decrease in molecular weight of the PHA during heating and/or drying of the PHA is reduced or prevented.

In an embodiment of the present invention, an aqueous suspension is provided which contains a polyhydroxyalkanoate and a dispersing agent and has a pH of not more than 7, the dispersing agent being an alkylene oxide-based dispersing agent.

A concentration of PHA in the aqueous PHA suspension obtained by the step (a) of the present production method is preferably not less than 30% by weight, more preferably not less than 40% by weight, and even more preferably not less than 50% by weight, from the viewpoint of (i) an economical advantage in terms of utility in drying and (ii) a resultant improvement in productivity. An upper limit of the concentration of the PHA is preferably not more than 65% by weight, more preferably not more than 60% by weight, because, otherwise, it may not be possible to ensure sufficient flowability due to closest packing. A method of adjusting the concentration of the PHA is not particularly limited. For example, the concentration can be adjusted by addition of an aqueous medium and/or by removal of part of an aqueous medium (for example, by centrifugal separation followed by removal of a supernatant). The adjustment of the concentration of the PHA can be carried out at any stage in the step (a), or can be carried out prior to the step (a).

In an embodiment of the present invention, the present production method is such that the aqueous suspension prepared in the step (a) contains the polyhydroxyalkanoate at a concentration of not less than 30% by weight and not more than 65% by weight.

A volume median diameter (hereinafter, simply referred to as a "volume median diameter of PHA") of PHA in the aqueous PHA suspension obtained by the step (a) of the present production method is preferably not more than 50 times, more preferably not more than 20 times, even more preferably not more than 10 times a volume median diameter (hereinafter referred to as a "primary particle size") of a primary particle of the PHA. In a case where a volume median diameter of the PHA is not less than 50 times the primary particle size, the aqueous PHA suspension exhibits an even more excellent flowability. This enables the subsequent step (b) to be carried out highly efficiently and thus tends to even more improve the productivity of the PHA.

In an embodiment of the present invention, PHA has a volume median diameter of, for example, preferably 0.5 $\mu$m to 5 $\mu$m, more preferably 1 $\mu$m to 4.5 $\mu$m, and even more preferably 1 $\mu$m to 4 $\mu$m, from the viewpoint of achieving an excellent flowability. A volume median diameter of PHA is measured by a method described in the Examples.

Note that the above-described volume median diameter of PHA can be used as an index of a state of dispersion of the PHA in the aqueous PHA suspension. A method of adjusting the above-described volume median diameter of PHA is not particularly limited, and can be a publicly known means (e.g. stirring). For example, an aqueous PHA suspension in which a state of dispersion has been disrupted by exposure to an acidic condition or the like (for example, in a case where the step (a2) is carried out before the step (a1)) can be subjected to a physical treatment, a chemical treatment, a biological treatment, or the like that can be arrived at by a person skilled in the art, to thereby regain the state of dispersion (for example, a state in which PHA has the above-described volume median diameter) of PHA in the aqueous PHA suspension.

(Step (b))

In the step (b) of the present production method, the aqueous PHA suspension prepared in the step (a) is spray-dried. Examples of a spray-drying method include a method in which the aqueous PHA suspension in a state of fine droplets is supplied into a dryer and dried in contact with hot air in the dryer. A method (atomizer) of supplying the aqueous PHA suspension in a state of fine droplets into a dryer is not particularly limited, and can be a publicly known method such as a method using a rotary disc or a method using a nozzle. A manner of contact between the droplets and the hot air in the dryer is not particularly limited. For example, the droplets and the hot air can be brought in contact with each other in a co-current manner, a countercurrent manner, or in a manner combining a co-current manner and a countercurrent manner.

A drying temperature in spray-drying of the step (b) may be any temperature at which most of the aqueous medium can be removed from the droplets of the aqueous PHA suspension. The drying temperature can be set as appropriate, provided that the aqueous PHA solution can be dried until a desired moisture content is achieved and that a deterioration in quality (a decrease in molecular weight, a reduction in color tone, and the like), melting, and the like are minimized. For example, a temperature of hot air to be blown into a spray dryer can be selected as appropriate within a range of 100°C to 300°C. A volume of hot air in the dryer can be set as appropriate, for example, in accordance

with a size of the dryer and the like.

The present production method can include, after the step (b), a step (for example, a step of subjecting PHA to drying under reduced pressure, or the like) of further drying resultant PHA (PHA powder or the like). The present production method can include other steps (for example, a step of adding various additives to the aqueous PHA suspension, or the like).

The present production method makes it possible to obtain PHA with high productivity. In particular, the present production method makes it possible to reduce cost (facility cost, utilities, and the like) of the drying step. Further, the present production method makes it possible to obtain PHA in a power state, and thus makes it possible to highly efficiently obtain PHA with excellent handleability.

[2. Polyhydroxyalkanoate powder]

Polyhydroxyalkanoate powder (hereinafter referred to as "the present PHA powder") in accordance with an embodiment of the present invention contains an alkylene oxide-based dispersing agent and has a bulk density of 0.3 kg/L to 0.5 kg/L and a median particle size of 80 μm to 200 μm.

In the present embodiment, for the "polyhydroxyalkanoate" and the "dispersing agent", reference is made to those described above.

A bulk density of the present PHA powder is not particularly limited, but is preferably 0.30 kg/L to 0.50 kg/L, more preferably 0.35 kg/L to 0.50 kg/L, and even more preferably 0.40 kg/L to 0.50 kg/L, from the viewpoint of achieving an excellent flowability. A bulk density of the present PHA powder is measured by a method described in the Examples.

A median particle size of the present PHA powder is not particularly limited, but is preferably 80 μm to 200 μm and more preferably 100 μm to 150 μm, from the viewpoint of achieving an excellent flowability. A median particle size of the present PHA powder is measured by a method described in the Examples.

The present PHA powder can contain the dispersing agent described above. An amount of the dispersing agent contained in the PHA powder is not particularly limited, but is preferably 0.1 parts by weight to 20 parts by weight, more preferably 0.5 parts by weight to 10 parts by weight, and even more preferably 0.75 parts by weight to 5 parts by weight relative to 100 parts by weight of the PHA contained in the PHA powder. In a case where an amount of the dispersing agent added is within the above range, productivity of the PHA powder tends to further improve.

The present PHA powder can contain various components produced or not removed during the present production method, provided that an effect of the present invention is exhibited.

The present PHA powder can be used for various applications such as paper, a film, a sheet, a tube, a plate, a rod, a container (e.g. a bottle container and the like), a bag, and a part.

[Embodiment 2]

[1. Method for producing PHA]

In Embodiment 2, only matters different from Embodiment 1 will be described. For the other matters, reference is made to the descriptions of Embodiment 1.

A method in accordance with an embodiment of the present invention for producing a polyhydroxyalkanoate includes the steps of: (a) preparing an aqueous suspension that contains a polyhydroxyalkanoate, an alkylene oxide-based dispersing agent, and a binder and has a pH of not more than 7; and (b) spray-drying the aqueous suspension prepared in the step (a). That is, the production method in accordance with the present embodiment includes the following steps (a) and (b) as essential steps.

- Step (a): preparing an aqueous suspension that contains PHA, an alkylene oxide-based dispersing agent, and a binder and has a pH of not more than 7
- Step (b): spray-drying the aqueous suspension prepared in the step (a)

(Step (a))

In the step (a) of the production method in accordance with the present embodiment, an aqueous suspension that contains PHA, an alkylene oxide-based dispersing agent, and a binder and has a pH of not more than 7 is prepared. In the aqueous suspension, the PHA is present dispersed in an aqueous medium, and the alkylene oxide-based dispersing agent and the binder are dissolved in the aqueous medium.

<PHA>

For this item, reference is made to the descriptions of Embodiment 1 except for the following matters.
The step (a) preferably includes the following steps (a1) and (a2).

- Step (a1): adding an alkylene oxide-based dispersing agent and a binder to an aqueous PHA suspension
- Step (a2): adjusting a pH of the aqueous PHA suspension to not more than 7

The order of carrying out the step (a1) and the step (a2) is not particularly limited, but it is preferable to carry out the step (a2) after the step (a1), from the viewpoint of reducing or preventing agglomeration of PHA in the step (a2) and obtaining an aqueous suspension that is even more excellent in dispersion stability of PHA.
In the step (a), an aqueous PHA suspension (an aqueous PHA suspension to which neither the alkylene oxide-based dispersing agent nor the binder has been added) used as a starting material is not particularly limited, and can be obtained, for example, by a method including a culture step of culturing a microorganism capable of producing PHA in a cell of the microorganism and a purification step of decomposing and/or removing a substance other than PHA after the culture step.
The production method in accordance with the present embodiment can include, before the step (a), a step (for example, a step including the culture step and the purification step described above) of obtaining an aqueous PHA suspension (an aqueous PHA suspension to which neither the alkylene oxide-based dispersing agent nor the binder has been added).
In an embodiment of the present invention, a shear viscosity of the aqueous PHA suspension is not particularly limited. From the viewpoint of achieving an excellent flowability, however, a viscosity of the aqueous PHA suspension at a shear rate of 100 1/s and at 20°C is preferably 0.001 Pa·s to 0.05 Pa·s, and more preferably 0.0015 Pa·s to 0.03 Pa·s. A shear viscosity of the aqueous PHA suspension is measured by a method described in the Examples.

<Alkylene oxide-based dispersing agent>

For this item, reference is made to the descriptions of Embodiment 1.

<Binder>

The binder in the step (a) of the production method in accordance with the present embodiment enables PHA powder after spray-drying to have a good flowability.
As used herein, the term "binder" refers to a substance that is capable of accelerating agglomeration of powdery and/or granular starting material during granulation to increase a granulation rate and thus increase a yield. The binder is not limited to any particular one, provided that an effect of the present invention is exhibited. Examples of the binder include modified cellulose, sodium polyacrylate, starch, dextrin, alginic acid, sodium alginate, chitosan, and gelatin. In an embodiment of the present invention, from the viewpoint of water-solubility, preferable examples of the binder include modified cellulose, sodium polyacrylate, dextrin, alginic acid, and sodium alginate.
As used herein, the term "modified cellulose" refers to cellulose in which at least a part of a hydroxy group has been substituted with a given substituent. Note that the "modified cellulose" may also be referred to as a "cellulose derivative".
In an embodiment of the present invention, the modified cellulose can preferably be cellulose in which at least a part of a hydroxy group has been substituted with a methoxy group and/or a hydroxypropoxy group.
In an embodiment of the present invention, the modified cellulose is not limited to any particular one, provided that an effect of the present invention is exhibited. Examples of the modified cellulose include methyl cellulose (MC), ethyl cellulose, propyl cellulose, hydroxymethyl cellulose, hydroxyethyl cellulose (HEC), hydroxypropyl cellulose, hydroxyethyl methyl cellulose, hydroxyethyl ethyl cellulose, hydroxypropyl methyl cellulose (HPMC), carboxymethyl cellulose (CMC), carboxyethyl cellulose, carboxypropyl cellulose, carboxymethyl hydroxyethyl cellulose, acetyl cellulose, cyanoethyl cellulose, and cellulose sodium sulfate. Among these examples, methyl cellulose and hydroxypropyl methyl cellulose are preferable for being water-soluble in a wide range of substitution degrees. These modified celluloses may be used alone or in combination of a plurality of kinds.
The binder used in the step (a) (in particular, the step (a1)) of the production method in accordance with the present embodiment is not particularly limited, and can be, for example, a commercially available binder. Examples of a commercially available modified cellulose include MCE-15 (manufactured by Shin-Etsu Chemical Co., Ltd.), MCE-100 (manufactured by Shin-Etsu Chemical Co., Ltd.), MCE-400 (manufactured by Shin-Etsu Chemical Co., Ltd.), MCE-4000 (manufactured by Shin-Etsu Chemical Co., Ltd.), SFE-400 (manufactured by Shin-Etsu Chemical Co., Ltd.), SFE-4000 (manufactured by Shin-Etsu Chemical Co., Ltd.), SE-50 (manufactured by Shin-Etsu Chemical Co., Ltd.), and NE-100

(manufactured by Shin-Etsu Chemical Co., Ltd.).

In an embodiment of the present invention, the binder is preferably a biodegradable substance, from the viewpoint of environmental problems.

An amount of the binder added to the aqueous PHA suspension in the step (a) (in particular, the step (a1)) of the production method in accordance with the present embodiment is not particularly limited, but is preferably 0.01 parts by weight to 10 parts by weight, more preferably 0.05 parts by weight to 5 parts by weight, and even more preferably 0.08 parts by weight to 3 parts by weight relative to 100 parts by weight of the PHA contained in the aqueous suspension. In a case where the amount of the binder added is within the above range, an effect of the present invention can be exhibited.

In an embodiment of the present invention, a shear viscosity of a 2 wt% aqueous binder solution is preferably not more than 1.0 Pa·s, more preferably not more than 0.8 Pa·s, and even more preferably not more than 0.5 Pa·s, from the viewpoint of achieving an excellent flowability. A lower limit value is not particularly set, but is preferably not less than 0.001 Pa·s, more preferably not less than 0.005 Pa·s, and even more preferably not less than 0.01 Pa·s.

[Others]

For this item, reference is made to the descriptions of Embodiment 1 except for the following matters.

The aqueous PHA suspension (the aqueous PHA suspension to which neither the alkylene oxide-based dispersing agent nor the binder has been added) that has not been subjected to the step (a) of the production method in accordance with the present embodiment ordinarily has a pH of more than 7 by being subjected to the above purification step.

In an embodiment of the present invention, an aqueous suspension is provided which contains a polyhydroxyalkanoate, an alkylene oxide-based dispersing agent, and a binder and has a pH of not more than 7.

As described in [Overview of the present invention], the inventors of the present invention found, while advancing their study, that in a case where a specific PHBH is used as an example of PHA, an aqueous suspension of the PHBH has a poor dispersion stability, and it thus becomes difficult to send the aqueous suspension to a spray dryer. Specifically, it was found that this problem is caused in a case where PHBH (hereinafter referred to as a "specific PHBH") in which a composition ratio of a 3-hydroxybutyrate unit to a 3-hydroxyhexanoate unit is 80/20 (mol/mol) to 91/9 (mol/mol). The inventors of the present invention then found that this problem is also solvable by addition of a binder to the aqueous PHA suspension.

Thus, in the present embodiment (that is, "an aqueous suspension that contains PHA, an alkylene oxide-based dispersing agent, and a binder and has a pH of not more than 7"), even in a case where the PHA is the specific PHBH, the aqueous suspension not only makes it possible to obtain PHA (e.g. PHA powder) having an excellent flowability but also is capable of retaining a good dispersion stability.

(Step (b))

For this item, reference is made to the descriptions of Embodiment 1 except for the following matters.

Further, according to the production method in accordance with the present embodiment, it is possible to obtain PHA (e.g. PHA powder) having an excellent flowability. Further, according to the production method in accordance with the present embodiment, even in a case where the specific PHBH is used, it is possible to obtain an aqueous PHA suspension that retains a good dispersion stability.

[2. Polyhydroxyalkanoate powder]

Polyhydroxyalkanoate powder in accordance with the present embodiment contains a polyhydroxyalkanoate, an alkylene oxide-based dispersing agent, and a binder.

In the present embodiment, for the "polyhydroxyalkanoate", the "alkylene oxide-based dispersing agent", and the "binder", reference is made to those described above.

The PHA powder in accordance with the present embodiment has a compressibility rate of, for example, less than 30%, preferably not more than 29.8%, more preferably not more than 29.6%, and even more preferably not more than 29.5%. In a case where the PHA powder in accordance with the present embodiment has a compressibility rate of less than 30%, a good flowability of the powder is achieved. The lower the compressibility rate, the better the flowability of the PHA powder. The compressibility rate is thus not limited to any particular lower limit value, but can be, for example, not less than 5%.

The present invention is not limited to the embodiments, but can be altered by a skilled person in the art within the scope of the claims. The present invention also encompasses, in its technical scope, any embodiment derived by combining technical means disclosed in differing embodiments.

In other words, an embodiment of the present invention is as follows.

<1> A method for producing a polyhydroxyalkanoate, including the steps of:

(a) preparing an aqueous suspension that contains a polyhydroxyalkanoate and an alkylene oxide-based dispersing agent and has a pH of not more than 7; and
(b) spray-drying the aqueous suspension prepared in the step (a).

<2> The method as set forth in claim 1, wherein the aqueous suspension prepared in the step (a) further contains a binder.
<3> The method as set forth in claim 2, wherein a 2 wt% aqueous binder solution of the binder has a shear viscosity of not more than 1.0 Pa·s.
<4> The method as set forth in any one of <1> through <3>, wherein the alkylene oxide-based dispersing agent is constituted by a block of poly(ethylene oxide), PEO, and a block of poly(propylene oxide), PPO, and is in a form of PEO-PPO-PEO.
<5> The method as set forth in any one of <1> through <4>, wherein a molecular weight of PEO in the alkylene oxide-based dispersing agent is not less than 1500, and a ratio of the molecular weight of PEO to a molecular weight of PPO in the dispersing agent is 0.5 to 5.0.
<6> The method as set forth in any one of <1> through <5>, wherein the alkylene oxide-based dispersing agent is a compound represented by the following formula (1):

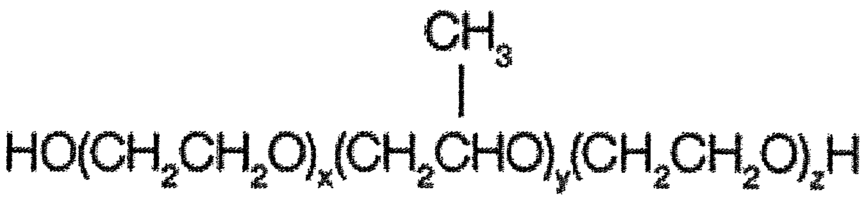

· · · (1)

where X is 17 to 340, Y is 8 to 115, and Z is 17 to 340.
<7> The method as set forth in any one of <1> through <6>, wherein the aqueous suspension prepared in the step (a) contains the polyhydroxyalkanoate at a concentration of not less than 30% by weight and not more than 65% by weight.
<8> The method as set forth in <2> or <3>, wherein the binder is one selected from the group consisting of modified cellulose, sodium polyacrylate, starch, dextrin, alginic acid, sodium alginate, chitosan, and gelatin.
<9> An aqueous suspension, containing: a polyhydroxyalkanoate; and an alkylene oxide-based dispersing agent, the aqueous suspension having a pH of not more than 7.
<10> The aqueous suspension as set forth in <9>, further containing a binder.
<11> The aqueous suspension as set forth in <10>, wherein the binder is one selected from the group consisting of modified cellulose, sodium polyacrylate, starch, dextrin, alginic acid, sodium alginate, chitosan, and gelatin.
<12> Hydroxyalkanoate powder, containing: a polyhydroxyalkanoate; and an alkylene oxide-based dispersing agent, the hydroxyalkanoate powder having a bulk density of 0.3 kg/L to 0.5 kg/L and a median particle size of 80 μm to 200 μm.
<13> Hydroxyalkanoate powder, containing: a polyhydroxyalkanoate; an alkylene oxide-based dispersing agent; and a binder.
<14> The hydroxyalkanoate powder as set forth in <13>, wherein the binder is one selected from the group consisting of modified cellulose, sodium polyacrylate, starch, dextrin, alginic acid, sodium alginate, chitosan, and gelatin.

Examples

The following description will discuss embodiments of the present invention in further detail on the basis of Examples. However, the present invention is not limited by the Examples. Note that, in the Examples, "PHBH" is used as "PHA", and "PHA" can be read as "PHBH".

[Measuring method and evaluating method]

In Examples and Comparative Examples, measurement and evaluation were carried out by the following methods.

(Volume median diameter)

A volume median diameter in an aqueous PHA suspension was measured with a laser diffraction/ scatter particle size distribution meter LA-950 manufactured by HORIBA.

(Bulk density)

In accordance with a method described in JIS K-7365, measurement was carried out using an apparatus including (i) a metal cylinder (receiver) which had a smoothened inner surface, a volume of 100 ml ± 0.5 ml, and an inner diameter of 45 mm ± 5 mm and (ii) a funnel which was set above the metal cylinder, had a lower opening of 20 mm to 30 mm, and had a damper (e.g. a metal plate) attached to the funnel. A scale capable of measuring weights in decigrams was used.

The specific measuring method is as follows. The funnel and the cylinder were held vertically so that the axes of the funnel and the cylinder coincided with each other. Prior to testing, the powder was mixed well. The damper at the lower opening of the funnel was closed, and 110 ml to 120 ml of the powder was introduced into the funnel. The damper was pulled off quickly so as to cause the material to flow into the receiver. When the receiver became full, part of the material, which part was protruding from the top of the receiver, was removed with use of a straight plate. The scale was used to weigh a mass of the content of the receiver to an accuracy of one decigram. The powder to be tested was subjected the above measurement twice.

An apparent bulk density (unit: kg/L) of the material thus tested was calculated by the following formula:

$$m / V$$

where m is a mass (g) of the content of the receiver and V is a volume (ml) (that is, 100) of the receiver. A number-based arithmetical mean of measured results from the two times of measurement was used as the result.

(Median particle size)

An average particle size of PHA powder after spray-drying, which PHA powder was obtained by the present production method, was measured by the following method.

The average particle size was measured with a laser diffraction/scatter particle size distribution meter LA-950 (HORIBA). The specific measuring method is as follows. To 20 ml of ion exchange water, 0.05 g of sodium dodecyl sulfate, which is a surfactant, was added as a dispersing agent to obtain an aqueous solution of the surfactant. Then, 0.2 g of resin particles to be subjected to measurement was added to the aqueous solution of the surfactant and dispersed in the aqueous solution of the surfactant to obtain a dispersion slurry for measurement. The dispersion slurry thus prepared was introduced into the laser diffraction/ scatter particle size distribution meter and subjected to measurement.

(Shear viscosity of aqueous PHA suspension)

With a rheometer AR-G2 (manufactured by TA Instrument), a shear viscosity of an aqueous PHA suspension was measured in a coaxial double cylinder. The specific measuring method is as follows. 20 mL of the aqueous PHA suspension was introduced into the cylinder and was cooled under the condition of a shear rate of 100 1/s until a liquid temperature was 15°C. In Examples 1 through 7 and Comparative Examples 1 through 3, the shear rate was changed to 10 1/s after the intended liquid temperature was reached, and a viscosity was measured when a change in torque over time had become less than 1%. In Examples 8 through 24 and Comparative Examples 4 through 7, after the intended liquid temperature was reached, the liquid temperature was increased to 20°C, and then a viscosity was measured when a change in torque over time had become less than 1%.

(Compressibility rate)

A compressibility rate was measured by a method by R. L. Carr (Carr, R. L., Chem. Eng., 72, 163 (1965)). A compressibility rate is defined by the following formula (2).

$$\text{Compressibility rate} = \{(\text{compacted bulk density}) - (\text{loose bulk density})\} / (\text{compacted bulk density}) \times 100 \ldots (2)$$

(Shear viscosity of 2 wt% aqueous binder solution)

With a rheometer AR-G2 (manufactured by TA Instrument), a shear viscosity of an 2 wt% aqueous binder solution was measured in a coaxial double cylinder. The specific measuring method is as follows. 20 mL of the aqueous PHA suspension was introduced into the cylinder and was cooled under the condition of a shear rate of 100 1/s until a liquid temperature was 15°C. After the intended liquid temperature was reached, the liquid temperature was increased to 20°C, and then a viscosity was measured when a change in torque over time had become less than 1%. The shear viscosity of the 2 wt% aqueous binder solution is shown in Table 1 below. Note that a 2 wt% aqueous binder solution of methyl cellulose (MCE-4000) and a 2 wt% aqueous binder solution of hydroxypropyl methyl cellulose (SFE-4000) each had an unmeasurably high shear viscosity.

[Table 1]

| Binder | Product name | Shear viscosity of 2wt% aqueous binder solution [Pa·s] |
|---|---|---|
| Methyl cellulose | MCE-15 | 0.018 |
| Methyl cellulose | MCE-100 | 0.1162 |
| Methyl cellulose | MCE-400 | 0.3679 |
| Methyl cellulose | MCE-4000 | Beyond measurable upper limit |
| Hydroxypropyl methyl cellulose | SFE-400 | 0.3493 |
| Hydroxypropyl methyl cellulose | SFE-4000 | Beyond measurable upper limit |
| Hydroxypropyl methyl cellulose | SE-50 | 0.0565 |
| Hydroxypropyl methyl cellulose | NE-100 | 0.1106 |

[Example 1]

(Preparation of bacterial cell culture solution)

*Ralstonia eutropha* KNK-005 strain indicated in paragraph [0049] of International Publication No. WO 2008/010296 was cultured by a method described in paragraphs [0050] to [0053] of the same document to obtain a bacterial cell culture solution containing bacterial cells containing PHA. Note that *Ralstonia eutropha* is currently classified as *Cupriavidus necator.* A composition ratio (a composition ratio of a 3-hydroxybutyrate unit to a 3-hydroxyhexanoate unit) of repeating units of PHA was 92/8 (mol/mol) to 99/1(mol/mol).

(Sterilization)

The obtained bacterial cell culture solution above was subjected to heating and stirring at an internal temperature of 60°C to 80°C for 20 minutes for sterilization.

(High-pressure disruption)

To the resultant bacterial cell culture solution which had been sterilized, 0.2% by weight of sodium dodecyl sulfate was added. Further, an aqueous sodium hydroxide solution was added to achieve a pH of 11.0, and then a resultant mixture was maintained at 50°C for one hour. Then, the mixture was subjected to high-pressure disruption at a pressure of 450 $kgf/cm^2$ to 550 $kgf/cm^2$ with a high-pressure disruptor (a high-pressure homogenizer model PA2K, manufactured by Niro-Soavi).

(Purification)

To the resultant disrupted solution which had been subjected to the high-pressure disruption, distilled water

was added in an amount equivalent to the disrupted solution. A solution thus obtained was centrifuged, and a supernatant was removed to concentrate the solution by two times. To the resultant concentrated aqueous suspension of PHA, an aqueous sodium hydroxide solution (pH: 11) was added in an amount equal to that of the removed supernatant. A resultant mixture was centrifuged, and a supernatant was removed. To this, water was added again to obtain a suspension, and 0.2 wt% sodium dodecyl sulfate and protease (Novozymes, Esperase) in an amount of 1/100th of the weight of the PHA were added. A resultant mixture was stirred for 2 hours while being maintained at pH 10 and 50°C. The mixture was then centrifuged, and a supernatant was removed to concentrate the mixture by four times. Further, water was added to adjust a concentration of the PHA to 52.8% by weight.

(Granulation)

To the resultant aqueous PHA suspension (solid content concentration: 52.8% by weight), a nonionic dispersing agent of an ethylene oxide/propylene oxide copolymer (a molecular weight of polyethylene oxide: 8000, a molecular weight of polypropylene oxide: 2000, a product name: Plonon 208) was added as a dispersing agent in an amount of 0.95 phr (0.95 parts by weight relative to 100 parts by weight of PHA that was present in the aqueous suspension). Then, the solid content concentration was adjusted to 50%. The resultant liquid was stirred for 120 minutes, and then 10 wt% sulfuric acid was added in an amount of 0.4 ml per 100 g of the aqueous PHA suspension. With a laser diffraction/scatter particle size distribution meter LA-950 manufactured by HORIBA, a volume median diameter in the aqueous PHA suspension was measured to be 3.9 μm. Further, with AR-G2 manufactured by TA Instrument, a shear viscosity of the aqueous PHA suspension was measured to be such that a viscosity of the aqueous PHA suspension at a shear rate of 10 1/s was 0.361 Pa·s. Thus, a suspension excellent in property of being sent was obtained. The aqueous PHA suspension thus obtained was subjected to spray-drying (a temperature of hot air: 150°C, a temperature of exhaust air: 105°C) with a spray dryer OC-16 manufactured by OHKAWARA. The resultant dried PHA powder had a median particle size of 107 μm and a bulk density of 0.48 kg/L.

Note that, a volume median diameter before the addition of 10wt% sulfuric acid was measured to be 2.5 μm.

[Example 2]

An aqueous PHA suspension was obtained by a method similar to that of Example 1, except that a dispersing agent (product name: Pluronic 10400) containing polyethylene oxide having a molecular weight of 2167 and polypropylene oxide having a molecular weight of 3250 was used as a nonionic dispersing agent of an ethylene oxide/propylene oxide copolymer. In the obtained aqueous suspension, PHA had a volume median diameter of 2.7 μm. A viscosity of the aqueous suspension at a shear rate of 10 1/s was 0.057 Pa·s. Dried PHA powder was obtained by a method similar to that of Example 1 and had a median particle size of 111 μm and a bulk density of 0.45 kg/L.

[Example 3]

An aqueous PHA suspension was obtained by a method similar to that of Example 1, except that a dispersing agent (product name: Epan U108) containing polyethylene oxide having a molecular weight of 13000 and polypropylene oxide having a molecular weight of 3250 was used as a nonionic dispersing agent of an ethylene oxide/propylene oxide copolymer. In the obtained aqueous suspension, PHA had a volume median diameter of 2.4 μm. A viscosity of the aqueous suspension at a shear rate of 10 1/s was 0.027 Pa·s. Dried PHA powder was obtained by a method similar to that of Example 1 and had a median particle size of 122 μm and a bulk density of 0.45 kg/L.

[Example 4]

An aqueous PHA suspension was obtained by a method similar to that of Example 1, except that a dispersing agent (product name: Pluronic 10500) containing polyethylene oxide having a molecular weight of 3250 and polypropylene oxide having a molecular weight of 3250 was used as a nonionic dispersing agent of an ethylene oxide/propylene oxide copolymer. In the obtained aqueous suspension, PHA had a volume median diameter of 2.6 μm. A viscosity of the aqueous suspension at a shear rate of 10 1/s was 0.034 Pa·s. Dried PHA powder was obtained by a method similar to that of Example 1 and had a median particle size of 125 μm and a bulk density of 0.46 kg/L.

[Example 5]

An aqueous PHA suspension was obtained by a method similar to that of Example 1, except that a dispersing agent (product name: Genapol PF80) containing polyethylene oxide having a molecular weight of 6720 and polypropylene oxide having a molecular weight of 1680 was used as a nonionic dispersing agent of an ethylene oxide/propylene oxide

copolymer. In the obtained aqueous suspension, PHA had a volume median diameter of 4.6 μm. A viscosity of the aqueous suspension at a shear rate of 10 1/s was 0.951 Pa·s. Dried PHA powder was obtained by a method similar to that of Example 1 and had a median particle size of 115 μm and a bulk density of 0.44 kg/L.

[Example 6]

An aqueous PHA suspension was obtained by a method similar to that of Example 1, except that a dispersing agent (product name: Unilube 70DP-600B) containing polyethylene oxide having a molecular weight of 7000 and polypropylene oxide having a molecular weight of 3000 was used as a nonionic dispersing agent of an ethylene oxide/propylene oxide copolymer. In the obtained aqueous suspension, PHA had a volume median diameter of 3.9 μm. A viscosity of the aqueous suspension at a shear rate of 10 1/s was 0.035 Pa·s. Dried PHA powder was obtained by a method similar to that of Example 1 and had a median particle size of 131 μm and a bulk density of 0.49 kg/L.

[Example 7]

An aqueous PHA suspension was obtained by a method similar to that of Example 1, except that a dispersing agent (product name: Unilube 70DP-950B) containing polyethylene oxide having a molecular weight of 9100 and polypropylene oxide having a molecular weight of 3900 was used as a nonionic dispersing agent of an ethylene oxide/propylene oxide copolymer. In the obtained aqueous suspension, PHA had a volume median diameter of 2.7 μm. A viscosity of the aqueous suspension at a shear rate of 10 1/s was 0.033 Pa·s. Dried PHA powder was obtained by a method similar to that of Example 1 and had a median particle size of 129 μm and a bulk density of 0.48 kg/L.

[Comparative Example 1]

An aqueous PHA suspension was obtained by a method similar to that of Example 1, except that a dispersing agent (product name: Pluronic 6400) containing polyethylene oxide having a molecular weight of 1167 and polypropylene oxide having a molecular weight of 1750 was used as a nonionic dispersing agent of an ethylene oxide/propylene oxide copolymer. In the obtained aqueous suspension, PHA had a volume median diameter of 3.9 μm. A viscosity of the aqueous suspension at a shear rate of 10 1/s was 16.810 Pa·s. Since the aqueous suspension had a high viscosity, it was not possible to carry out spray-drying.

[Comparative Example 2]

An aqueous PHA suspension was obtained by a method similar to that of Example 1, except that a dispersing agent (product name: Epan 785) containing polyethylene oxide having a molecular weight of 11333 and polypropylene oxide having a molecular weight of 2000 was used as a nonionic dispersing agent of an ethylene oxide/propylene oxide copolymer. In the obtained aqueous suspension, PHA had a volume median diameter of 4.9 μm. A viscosity of the aqueous suspension at a shear rate of 10 1/s was 4.565 Pa·s. Since the aqueous suspension had a high viscosity, it was not possible to carry out spray-drying.

[Comparative Example 3]

An aqueous PHA suspension was obtained by a method similar to that of Example 1, except that a dispersing agent (product name: Pluronic 10100) containing polyethylene oxide having a molecular weight of 361 and polypropylene oxide having a molecular weight of 3250 was used as a nonionic dispersing agent of an ethylene oxide/propylene oxide copolymer. In the obtained aqueous suspension, PHA had a volume median diameter of 7.0 μm. The aqueous suspension had an unmeasurably high shear viscosity. Since the aqueous suspension had a high viscosity, it was not possible to carry out spray-drying.

Table 2 shows results of Examples 1 through 7 and Comparative Examples 1 through 3.

[Table 2]

| | Dispersing agent | Total molecular weight | Amount of EO [g/mol] | Amount of PO [g/mol] | Amount of EO/amount of PO | Shear viscosity [Pa·s] | Volume median diameter [μm] |
|---|---|---|---|---|---|---|---|
| Example 1 | Plonon 208 | 10000 | 8000 | 2000 | 4.0 | 0.361 | 3.9 |

(continued)

| | Dispersing agent | Total molecular weight | Amount of EO [g/mol] | Amount of PO [g/mol] | Amount of EO/amount of PO | Shear viscosity [Pa·s] | Volume median diameter [μm] |
|---|---|---|---|---|---|---|---|
| Example 2 | Pluronic 10400 | 5417 | 2167 | 3250 | 0.7 | 0.057 | 2.7 |
| Example 3 | Epan U108 | 16250 | 13000 | 3250 | 4.0 | 0.027 | 2.4 |
| Example 4 | Pluronic 10500 | 6500 | 3250 | 3250 | 1.0 | 0.034 | 2.6 |
| Example 5 | Genapol PF80 | 8400 | 6720 | 1680 | 4.0 | 0.951 | 4.6 |
| Example 6 | Unilube 70DP-600B | 10000 | 7000 | 3000 | 2.3 | 0.035 | 3.9 |
| Example 7 | Unilube 70DP-950B | 13000 | 9100 | 3900 | 2.3 | 0.033 | 2.7 |
| Comparative Example 1 | Pluronic 6400 | 2900 | 1160 | 1740 | 0.7 | 16.810 | 3.9 |
| Comparative Example 2 | Epan 785 | 13330 | 11333 | 2000 | 5.7 | 4.565 | 4.9 |
| Comparative Example 3 | Pluronic 10100 | 3500 | 350 | 3150 | 0.1 | Unmeasurable | 7.0 |

[Result 1]

Tabel 2 indicates that each of the aqueous

suspensions of Examples 1 through 7 had a viscosity lower than those of Comparative Examples 1 through 3. Further, Fig. 1 indicates that an aqueous suspension had a low viscosity only in a case where an amount of EO in a dispersing agent contained in the aqueous suspension and a ratio of the amount of the EO to an amount of PO in the dispersing agent were within specific ranges.

Thus, it was found that the present production method makes it possible to produce PHA (e.g. PHA powder) with high productivity.

[Example 8]

An aqueous PHA suspension having a solid content concentration of 52.8% by weight was prepared by carrying out the same operations as those of Example 1, up to purification. Subsequently, water was added to the aqueous PHA suspension to adjust the concentration (a solid content concentration (a concentration of PHA) of 32.8% by weight). To this, a nonionic dispersing agent of an ethylene oxide/propylene oxide copolymer (a molecular weight of polyethylene oxide: 8000, a molecular weight of polypropylene oxide: 2000, a product name: Plonon 208) was added in an amount of 1.0 phr (1 part by weight relative to 100 parts by weight of PHA that was present in the aqueous suspension). Then, methyl cellulose (product name: MCE-4000) was added as a binder in an amount of 1.0 phr, and a resultant mixture was mixed. Then, a solid content concentration of the mixture was adjusted to 30% by mass. The resultant liquid was stirred for 120 minutes, and then 10 wt% sulfuric acid was added in an amount of 0.4 ml per 100 g of the aqueous PHA suspension to obtain an aqueous PHA suspension. With a laser diffraction/ scatter particle size distribution meter LA-950 manufactured by HORIBA, a volume median diameter in this aqueous PHA suspension was measured to be 3.0 μm. Further, with AR-G2 manufactured by TA Instrument, a shear viscosity of the aqueous PHA suspension was measured to be such that a viscosity of the aqueous PHA suspension at a shear rate of 100 1/s at 20°C was 0.0129 Pa·s. The aqueous PHA suspension thus obtained was subjected to spray-drying (a temperature of hot air: 140°C, a temperature of exhaust air: 85°C, a rotation speed of a rotary atomizer: 10000 rpm) with a rotary atomizer-type spray dryer

(Mobile Minor) manufactured by GEA to obtain PHA powder. The obtained PHA powder had a compressibility rate of 21.3%.

[Example 9]

An aqueous PHA suspension was obtained by a method similar to that of Example 8, except that methyl cellulose (product name: MCE-400) was used as a binder. A viscosity of the obtained aqueous suspension at a shear rate of 100 1/s at 20°C was 0.0063 Pa·s. Further, the aqueous suspension was spray-dried by a method similar to that of Example 1 to obtain PHA powder. The obtained PHA powder had a compressibility rate of 27.5%.

[Example 10]

An aqueous PHA suspension was obtained by a method similar to that of Example 8, except that methyl cellulose (product name: MCE-100) was used as a binder. A viscosity of the obtained aqueous suspension at a shear rate of 100 1/s at 20°C was 0.0047 Pa·s. Further, the aqueous suspension was spray-dried by a method similar to that of Example 1 to obtain PHA powder. The obtained PHA powder had a compressibility rate of 29.4%.

[Example 11]

An aqueous PHA suspension was obtained by a method similar to that of Example 8, except that hydroxypropyl methyl cellulose (product name: SFE-4000) was used as a binder. A viscosity of the obtained aqueous suspension at a shear rate of 100 1/s at 20°C was 0.0107 Pa·s. Further, the aqueous suspension was spray-dried by a method similar to that of Example 1 to obtain PHA powder. The obtained PHA powder had a compressibility rate of 26.3%.

[Example 12]

An aqueous PHA suspension was obtained by a method similar to that of Example 8, except that hydroxypropyl methyl cellulose (product name: SFE-400) was used as a binder. A viscosity of the obtained aqueous suspension at a shear rate of 100 1/s at 20°C was 0.0061 Pa·s. Further, the aqueous suspension was spray-dried by a method similar to that of Example 1 to obtain PHA powder. The obtained PHA powder had a compressibility rate of 26.3%.

[Example 13]

An aqueous PHA suspension was obtained by a method similar to that of Example 8, except that methyl cellulose (product name: MCE-4000) was added in an amount of 0.8 phr. A viscosity of the obtained aqueous suspension at a shear rate of 100 1/s at 20°C was 0.0077 Pa·s. Further, the aqueous suspension was spray-dried by a method similar to that of Example 1 to obtain PHA powder. The obtained PHA powder had a compressibility rate of 25.0%.

[Example 14]

An aqueous PHA suspension was obtained by a method similar to that of Example 8, except that methyl cellulose (product name: MCE-4000) was added in an amount of 0.5 phr. A viscosity of the obtained aqueous suspension at a shear rate of 100 1/s at 20°C was 0.0049 Pa·s. Further, the aqueous suspension was spray-dried by a method similar to that of Example 1 to obtain PHA powder. The obtained PHA powder had a compressibility rate of 24.7%.

[Example 15]

An aqueous PHA suspension was obtained by a method similar to that of Example 8, except that methyl cellulose (product name: MCE-4000) was added in an amount of 0.3 phr. A viscosity of the obtained aqueous suspension at a shear rate of 100 1/s at 20°C was 0.0033 Pa·s. Further, the aqueous suspension was spray-dried by a method similar to that of Example 1 to obtain PHA powder. The obtained PHA powder had a compressibility rate of 25.0%.

[Example 16]

An aqueous PHA suspension was obtained by a method similar to that of Example 8, except that methyl cellulose (product name: MCE-4000) was added in an amount of 0.1 phr. A viscosity of the obtained aqueous suspension at a shear rate of 100 1/s at 20°C was 0.0020 Pa·s. Further, the aqueous suspension was spray-dried by a method similar to that of Example 1 to obtain PHA powder. The obtained PHA powder had a compressibility rate of 25.0%.

[Comparative Example 4]

An aqueous PHA suspension having a solid content concentration of 52.8% by weight was prepared by carrying out the same operations as those of Example 1, up to purification. Subsequently, water was added to the aqueous PHA suspension to adjust the concentration (a solid content concentration of 32.8% by weight). To this, a nonionic dispersing agent of an ethylene oxide/propylene oxide copolymer (a molecular weight of polyethylene oxide: 8000, a molecular weight of polypropylene oxide: 2000, a product name: Plonon 208) was added in an amount of 1.0 phr, and a resultant mixture was mixed. Then, a solid content concentration of the resultant mixture was adjusted to 30% by mass. The resultant liquid was stirred for 120 minutes, and then 10 wt% sulfuric acid was added in an amount of 0.4 ml per 100 g of the aqueous PHA suspension to obtain an aqueous PHA suspension. By a method similar to that of Example 1, a volume median diameter in the aqueous PHA suspension and a shear viscosity of the aqueous PHA suspension were measured. As a result, a viscosity of the aqueous PHA suspension at a shear rate of 100 1/s at 20°C was 0.0036 Pa·s. The obtained aqueous suspension was spray-dried by a method similar to that of Example 1 to obtain PHA powder. The obtained PHA powder had a compressibility rate of 30.0%.

Table 3 shows results of Examples 8 through 16 and Comparative Example 4.

[Table 3]

| | Binder | Product name | Shear viscosity of aqueous PHA suspension [Pa·s] | Concentration of binder [phr] | Concentration of dispersing agent [phr] | Compressibility rate [%] |
|---|---|---|---|---|---|---|
| Example 8 | Methyl cellulose | MCE-4000 | 0.0129 | 1.0 | 1.0 | 21.3 |
| Example 9 | Methyl cellulose | MCE-400 | 0.0063 | 1.0 | 1.0 | 27.5 |
| Example 10 | Methyl cellulose | MCE-100 | 0.0047 | 1.0 | 1.0 | 29.4 |
| Example 11 | Hydroxypropyl methyl cellulose | SFE-4000 | 0.0107 | 1.0 | 1.0 | 26.3 |
| Example 12 | Hydroxypropyl methyl cellulose | SFE-400 | 0.0061 | 1.0 | 1.0 | 26.3 |
| Example 13 | Methyl cellulose | MCE-4000 | 0.0077 | 0.8 | 1.0 | 25.0 |
| Example 14 | Methyl cellulose | MCE-4000 | 0.0049 | 0.5 | 1.0 | 24.7 |
| Example 15 | Methyl cellulose | MCE-4000 | 0.0033 | 0.3 | 1.0 | 25.0 |
| Example 16 | Methyl cellulose | MCE-4000 | 0.0020 | 0.1 | 1.0 | 25.0 |
| Comparative Example 4 | - | - | 0.0036 | - | 1.0 | 30.0 |

[Result 2]

Tabel 3 indicates that the PHA powders in Examples

8 through 16 each had a compressibility rate lower than that of the PHA powder in Comparative Example 4. That is, it was found that, in Examples 8 through 16, a flowability of powder was better than that in Comparative Example

4, in which no modified cellulose was contained. Further, from Examples 8 through 10, it was found that the higher a thickening property of an aqueous PHA suspension (the higher a thickening property of modified cellulose which is used), the better a flowability of a body. Further, from Examples 13 through 16, it was found that the higher a thickening property of an aqueous PHA suspension (the higher a thickening property of modified cellulose which is used), the better a flowability of powder even in a case where the modified cellulose is added in a small amount.

[Example 17]

An aqueous PHA suspension having a solid content concentration of 52% was prepared by carrying out the same operations as those of Example 1, except that a composition ratio (a composition ratio of a 3-hydroxybutyrate unit to a 3-hydroxyhexanoate unit) of repeating units of PHA was 80/20 (mol/mol) to 91/9 (mol/mol). Subsequently, to the aqueous PHA suspension (a solid content concentration of 52% by weight), a nonionic dispersing agent of an ethylene oxide/propylene oxide copolymer (a molecular weight of polyethylene oxide: 8000, a molecular weight of polypropylene oxide: 2000, a product name: Plonon 208) was added in an amount of 1.0 phr. Then, methyl cellulose (product name: MCE-15) was added in an amount of 0.3 phr, and a resultant mixture was mixed. Then, a solid content concentration of the resultant mixture was adjusted to 50% by mass. The resultant liquid was stirred for 120 minutes, and then 10 wt% sulfuric acid was added in an amount of 0.4 ml per 100 g of the aqueous PHA suspension to obtain an aqueous PHA suspension. In the obtained aqueous PHA suspension, a volume median diameter was 2.9 μm.

[Example 18]

An aqueous PHA suspension was obtained by a method similar to that of Example 17, except that methyl cellulose (product name: MCE-100) was used as a binder. In the obtained aqueous suspension, PHA had a volume median diameter of 2.4 μm.

[Example 19]

An aqueous PHA suspension was obtained by a method similar to that of Example 17, except that methyl cellulose (product name: MCE-400) was used as a binder. In the obtained aqueous suspension, PHA had a volume median diameter of 2.5 μm.

[Example 20]

An aqueous PHA suspension was obtained by a method similar to that of Example 17, except that methyl cellulose (product name: MCE-4000) was used as a binder. In the obtained aqueous suspension, PHA had a volume median diameter of 2.4 μm.

[Example 21]

An aqueous PHA suspension was obtained by a method similar to that of Example 17, except that hydroxypropyl methyl cellulose (product name: SFE-400) was used as a binder. In the obtained aqueous suspension, PHA had a volume median diameter of 2.5 μm.

[Example 22]

An aqueous PHA suspension was obtained by a method similar to that of Example 17, except that hydroxypropyl methyl cellulose (product name: SFE-4000) was used as a binder. In the obtained aqueous suspension, PHA had a volume median diameter of 2.5 μm.

[Example 23]

An aqueous PHA suspension was obtained by a method similar to that of Example 17, except that hydroxypropyl methyl cellulose (product name: SE-50) was used as a binder. In the obtained aqueous suspension, PHA had a volume median diameter of 2.6 μm.

[Example 24]

An aqueous PHA suspension was obtained by a method similar to that of Example 17, except that hydroxypropyl

methyl cellulose (product name: NE-100) was used as a binder. In the obtained aqueous suspension, PHA had a volume median diameter of 2.7 μm.

[Comparative Example 5]

An aqueous PHA suspension was obtained by a method similar to that of Example 17, except that (i) methyl cellulose (product name: MCE-4000) was used as a binder and (ii) no nonionic dispersing agent of an ethylene oxide/propylene oxide copolymer (a molecular weight of polyethylene oxide: 8000, a molecular weight of polypropylene oxide: 2000, a product name: Plonon 208) was added. In the obtained aqueous suspension, PHA had a volume median diameter of 4.8 μm.

[Comparative Example 6]

An aqueous PHA suspension was obtained by a method similar to that of Example 17, except that no binder was added. In the obtained aqueous suspension, PHA had a volume median diameter of 13.9 μm.

(Comparative Example 7)

An aqueous PHA suspension was obtained by a method similar to that of Comparative Example 6, except that (i) a nonionic dispersing agent of an ethylene oxide/propylene oxide copolymer (a molecular weight of polyethylene oxide: 8000, a molecular weight of polypropylene oxide: 2000, a product name: Plonon 208) was added in an amount of 1.3 phr and (ii) no binder was added. In the obtained aqueous suspension, PHA had a volume median diameter of 3.8 μm.

<Reference Example 1>

An aqueous PHA suspension was obtained by a method similar to that of Example 1, except that (i) a composition ratio of repeating units of PHA was 80/20 (mol/mol) to 91/9 (mol/mol) and (ii) none of a binder, a dispersing agent, and 10 wt% sulfuric acid was added. In the obtained aqueous suspension, PHA had a volume median diameter of 2.5 μm.
Table 4 shows results of Examples 17 through 24,
**[0164]** Comparative Examples 5 through 7, and Reference Example 1.

[Table 4]

| | Binder | Product name | Concentration of binder [phr] | Concentration of dispersing agent [phr] | Volume median diameter of PHA [μm] |
|---|---|---|---|---|---|
| Example 17 | Methyl cellulose | MCE-15 | 0.3 | 1.0 | 2.9 |
| Example 18 | Methyl cellulose | MCE-100 | 0.3 | 1.0 | 2.4 |
| Example 19 | Methyl cellulose | MCE-400 | 0.3 | 1.0 | 2.5 |
| Example 20 | Methyl cellulose | MCE-4000 | 0.3 | 1.0 | 2.4 |
| Example 21 | Hydroxypropyl methyl cellulose | SFE-400 | 0.3 | 1.0 | 2.5 |
| Example 22 | Hydroxypropyl methyl cellulose | SFE-4000 | 0.3 | 1.0 | 2.5 |
| Example 23 | Hydroxypropyl methyl cellulose | SE-50 | 0.3 | 1.0 | 2.6 |
| Example 24 | Hydroxypropyl methyl cellulose | NE-100 | 0.3 | 1.0 | 2.7 |
| Comparative Example 5 | Methyl cellulose | MCE-4000 | 0.3 | - | 4.8 |

(continued)

| | Binder | Product name | Concentration of binder [phr] | Concentration of dispersing agent [phr] | Volume median diameter of PHA [μm] |
|---|---|---|---|---|---|
| Comparative Example 6 | - | - | - | 1.0 | 13.9 |
| Comparative Example 7 | - | - | - | 1.3 | 3.8 |
| Reference Example 6 | - | - | - | - | 2 . 5 |

[Result 3]

Table 4 indicates that PHAs in the aqueous suspensions in Examples 17 through 24 each had a volume median diameter smaller than those in Comparative Examples 5 through 7. That is, it was found that, in Examples 17 through 24, since an alkylene oxide-based dispersing agent and a binder are contained, it is possible to retain a good dispersion stability in a case where a composition ratio of repeating units of PHA is 80/20 (mol/mol) to 91/9 (mol/mol).

Industrial Applicability

The present production method makes it possible to produce PHA (e.g. PHA powder) with high productivity, and thus can be advantageously used in production of PHA. Further, PHA powder and the like obtained by the present production method can be suitably used in fields such as agriculture, fishing, forestry, horticulture, medicine, sanitary products, clothing, non-clothing, packaging, automobiles, building materials, and the like.

## Claims

1. A method for producing a polyhydroxyalkanoate, comprising the steps of:

   (a) preparing an aqueous suspension that contains a polyhydroxyalkanoate and an alkylene oxide-based dispersing agent and has a pH of not more than 7; and
   (b) spray-drying the aqueous suspension prepared in the step (a).

2. The method as set forth in claim 1, wherein the aqueous suspension prepared in the step (a) further contains a binder.

3. The method as set forth in claim 2, wherein a 2 wt% aqueous binder solution of the binder has a shear viscosity of not more than 1.0 Pa·s.

4. The method as set forth in any one of claims 1 through 3, wherein the alkylene oxide-based dispersing agent is constituted by a block of poly(ethylene oxide), PEO, and a block of poly(propylene oxide), PPO, and is in a form of PEO-PPO-PEO.

5. The method as set forth in any one of claims 1 through 4, wherein a molecular weight of PEO in the alkylene oxide-based dispersing agent is not less than 1500, and a ratio of the molecular weight of the PEO to a molecular weight of PPO in the dispersing agent is 0.5 to 5.0.

6. The method as set forth in any one of claims 1 through 5, wherein the alkylene oxide-based dispersing agent is a compound represented by the following formula (1):

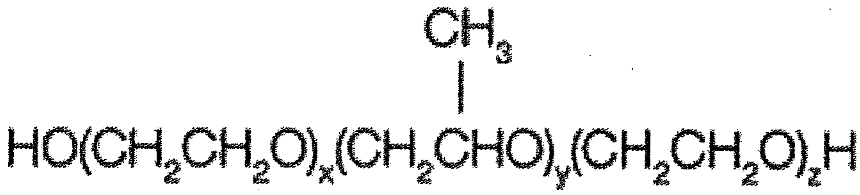

・・・(1)

where X is 17 to 340, Y is 8 to 115, and Z is 17 to 340.

**7.** The method as set forth in any one of claims 1 through 6, wherein the aqueous suspension prepared in the step (a) contains the polyhydroxyalkanoate at a concentration of not less than 30% by weight and not more than 65% by weight.

**8.** The method as set forth in claim 2 or 3, wherein the binder is one selected from the group consisting of modified cellulose, sodium polyacrylate, starch, dextrin, alginic acid, sodium alginate, chitosan, and gelatin.

**9.** An aqueous suspension, comprising:

a polyhydroxyalkanoate; and
an alkylene oxide-based dispersing agent,
the aqueous suspension having a pH of not more than 7.

**10.** The aqueous suspension as set forth in claim 9, further comprising a binder.

**11.** The aqueous suspension as set forth in claim 10, wherein the binder is one selected from the group consisting of modified cellulose, sodium polyacrylate, starch, dextrin, alginic acid, sodium alginate, chitosan, and gelatin.

**12.** Hydroxyalkanoate powder, comprising:

a polyhydroxyalkanoate; and
an alkylene oxide-based dispersing agent,
the hydroxyalkanoate powder having a bulk density of 0.3 kg/L to 0.5 kg/L and a median particle size of 80 μm to 200 μm.

**13.** Hydroxyalkanoate powder, comprising:

a polyhydroxyalkanoate;
an alkylene oxide-based dispersing agent; and
a binder.

**14.** The hydroxyalkanoate powder as set forth in claim 13, wherein the binder is one selected from the group consisting of modified cellulose, sodium polyacrylate, starch, dextrin, alginic acid, sodium alginate, chitosan, and gelatin.

FIG. 1

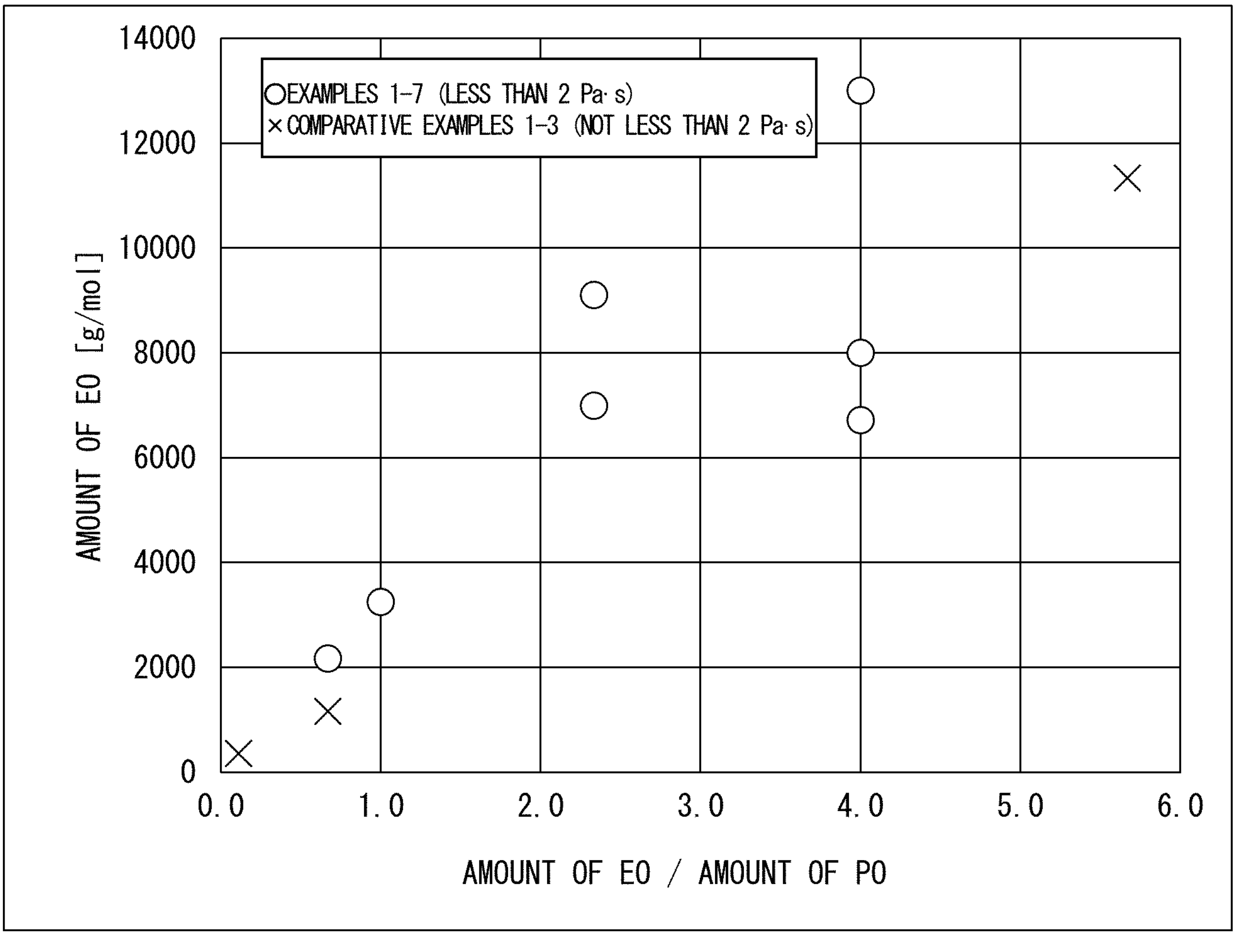

○EXAMPLES 1-7 (LESS THAN 2 Pa·s)
×COMPARATIVE EXAMPLES 1-3 (NOT LESS THAN 2 Pa·s)
AMOUNT OF EO [g/mol]
14000
12000
10000
8000
6000
4000
2000
0
0.0
1.0
2.0
3.0
4.0
5.0
6.0
AMOUNT OF EO / AMOUNT OF PO

**INTERNATIONAL SEARCH REPORT**

International application No.
PCT/JP2020/040643

A. CLASSIFICATION OF SUBJECT MATTER
B01F17/42(2006.01)i; C08J3/12(2006.01)i; C08L67/00(2006.01)i; C08L101/00(2006.01)i; C08L71/02(2006.01)i; C12P7/62(2006.01)i
FI: C08J3/12 101; B01F17/42; C08L67/00; C08L71/02; C08L101/00; C08J3/12 CFD; C12P7/62
According to International Patent Classification (IPC) or to both national classification and IPC

B. FIELDS SEARCHED
Minimum documentation searched (classification system followed by classification symbols)
B01F17/42; C08J3/12; C08L67/00; C08L101/00; C08L71/02; C12P7/62

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| Y | WO 2018/070492 A1 (KANEKA CORP.) 19 April 2018 (2018-04-19) claims, examples | 1-14 |
| Y | JP 2000-502375 A (MONSANTO COMPANY) 29 February 2000 (2000-02-29) claims, page 11, line 1 to bottom line, examples | 1-14 |
| Y | JP 1-203223 A (IDEMITSU KOSAN CO., LTD.) 16 August 1989 (1989-08-16) claims, page 2, lower right column, lines 1-7, examples | 2-3, 8, 10-11, 13-14 |
| A | JP 2007-308497 A (L'OREAL) 29 November 2007 (2007-11-29) paragraph [0031] | 1-14 |
| A | JP 8-502088 A (ZENECA LIMITED) 05 March 1996 (1996-03-05) entire text | 1-14 |

☐ Further documents are listed in the continuation of Box C.   ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 December 2020 (17.12.2020) | 28 December 2020 (28.12.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/040643

| Patent Documents referred in the Report | Publication Date | Patent Family | Publication Date |
|---|---|---|---|
| WO 2018/070492 A1 | 19 Apr. 2018 | EP 3527609 A1 claims, examples | |
| JP 2000-502375 A | 29 Feb. 2000 | WO 1997/021762 A1 claims, page 5, line 21 to page 6, line 4, examples | |
| JP 1-203223 A | 16 Aug. 1989 | (Family: none) | |
| JP 2007-308497 A | 29 Nov. 2007 | US 2007/0269390 A1 paragraph [0038] | |
| JP 8-502088 A | 05 Mar. 1996 | US 5599891 A entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018070492 A
- JP 5093049 A
- WO 2010067543 A
- WO 2008010296 A